(19) Europäisches Patentamt
European Patent Office
Office européen des brevets

(11) Veröffentlichungsnummer: **0 182 738 B1**

# (12) EUROPÄISCHE PATENTSCHRIFT

(45) Veröffentlichungstag der Patentschrift: **08.04.92**

(51) Int. Cl.5: **C07D 207/34**, C07D 207/33, C07B 43/08, A01N 43/36, C07C 49/255, //C07C43/176

(21) Anmeldenummer: **85810465.6**

(22) Anmeldetag: **10.10.85**

Die Akte enthält technische Angaben, die nach dem Eingang der Anmeldung eingereicht wurden und die nicht in dieser Patentschrift enthalten sind.

## (54) Verfahren zur Herstellung fungizid aktiver 4-Phenyl-pyrrol-Derivate.

(30) Priorität: **16.10.84 CH 4950/84**

(43) Veröffentlichungstag der Anmeldung:
**28.05.86 Patentblatt 86/22**

(45) Bekanntmachung des Hinweises auf die Patenterteilung:
**08.04.92 Patentblatt 92/15**

(84) Benannte Vertragsstaaten:
**AT BE CH DE FR GB IT LI LU NL SE**

(56) Entgegenhaltungen:
**DE-A- 2 927 480**

**PATENT ABSTRACTS OF JAPAN, Band 6, Nr. 122, (C-112)(1000), 7. Juli 1982; & JP - A - 57 50 953**

**PATENT ABSTRACTS OF JAPAN, Band 2, Nr. 93, 29. Juli 1978, Seite 1678C78; & JP - A - 53 59 645**

(73) Patentinhaber: **CIBA-GEIGY AG**
**Klybeckstrasse 141**
**CH-4002 Basel(CH)**

(72) Erfinder: **Martin, Pierre, Dr.**
**Meisenweg 38**
**CH-4310 Rheinfelden(CH)**
Erfinder: **Lang, Robert Werner, Dr.**
**Hagenbachweg 10**
**CH-4133 Pratteln(CH)**

EP 0 182 738 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft eine neues Verfahren Zur Herstellung von 4-Phenyl-pyrrol-derivaten der Formel I

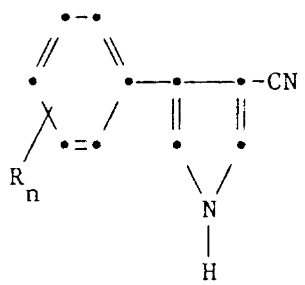

(I)

worin
R für Halogen, $C_1$-$C_6$-Alkyl oder $C_1$-$C_6$-Haloalkyl steht; und
n für 0, 1 oder 2 steht.

Unter dem Begriff Alkyl selbst als Bestandteil eines anderen Substituenten, wie Haloalkyl etc., sind je nach Zahl der angegebenen Kohlenstoffatome beispielsweise die folgenden geradkettigen oder verzweigten Gruppen zu verstehen: Methyl, Ethyl, Propyl, Butyl, Pentyl, Hexyl usw. sowie ihre Isomeren, wie z.B. Isopropyl, Isobutyl, tert.-Butyl, Isopentyl usw. Die Vorsilbe Halo in der Bezeichnung eines Substituenten bedeutet hier und im folgenden, dass dieser Substituent einfach bis perhalogeniert auftreten kann. Halogen und Halo stehen stellvertretend insbesondere für Fluor, Chlor oder Brom. Haloalkyl steht somit für einen einfach bis perhalogenierten Alkylrest, wie z.B. $CHCl_2$, $CH_2F$, $CCl_3$, $CH_2Cl$, $CHF_2$, $CH_2CH_2Br$, $C_2Cl_5$, $CHBr_2$, $CHBrCl$ usw., vorzugsweise für $CF_3$.

4-Phenyl-pyrrol-derivate der Formel I, worin n für 0, 1 oder 2 steht und der Pyrrol-Stickstoff unsubstituiert oder durch Acetyl substituiert ist, sind aus der DE-A 29 27 480 als Pflanzenfungizide bekannt.

In der DE-A 29 27 480 wird ein aus Tetrahedron Letters No. 52, S. 5337-5340, 1972 bekanntes Verfahren zur Herstellung von 4-Phenyl-3-cyanopyrrol-derivaten angegeben. Bei diesem als TosMIC-Verfahren bekannten Prozess wird ein Zimtsäurenitril der Formel X

(X)

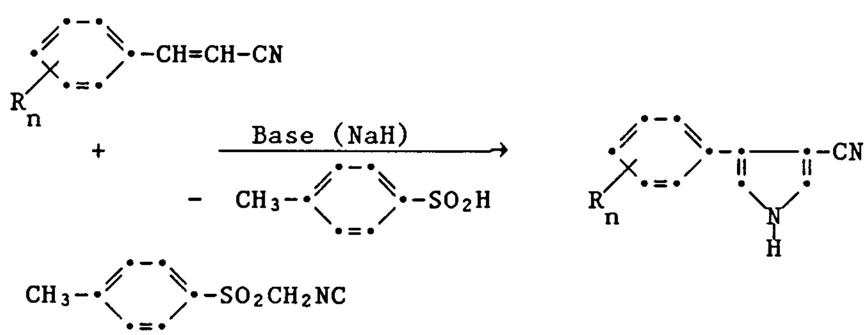


(XXX)

(XX)

mit Tosylmethylisocyanid (XX) [TosMIC] in Gegenwart einer starken Base, wie z.B. Natriumhydrid, zu 4-Phenyl-3-cyanopyrrolderivaten der Formel (XXX) cyclisiert; dabei hat R die unter Formel I angegebenen Bedeutungen und n steht für 0, 1 oder 2.

Obwohl eine Fülle von Pyrrolsynthesen bekannt ist (vgl. J.M. Patterson, Synthesis 1976, s. 281-304), führte bisher lediglich das oben skizzierte TosMIC-Verfahren unmittelbar zu den in 2- und 5-Stellung unsubstituierten, fungizid wertvollen 4-Phenyl-3-cyanopyrrol-derivaten. Allerdings wird in Tetrahedron Letters No. 52, S. 5337-5340, 1972 für die Herstellung des Grundkörpers 4-Phenyl-3-cyanopyrrol die für technische Zwecke geringe Ausbeute von nur 35 % angegeben. Es hat sich auch gezeigt, dass das Reagenz TosMIC für technische Synthesen schwerwiegende Nachteile aufweist. So neigt TosMIC bei erhöhten Temperaturen, insbesondere oberhalb von 90 ° C (übliche Trocknungsbedingungen) zu explosionsartigem Zerfall. Andererseits verbraucht eine Restfeuchtigkeit einen Teil der eingesetzten Base (Hydrolysegefahr/Ausbeutereduktion). Ferner zeigt TosMIC physiologische Nachteile wie starke Augen- und Hautirritationen.

Durch die genannten Nachteile erweist sich, das an sich brauchbare Laborverfahren, für die industrielle Produktion von 4-Phenyl-pyrrol-derivaten als ungeeignet.

In JP-A-57 50 953 und JP-A-53 59 645 wird die Umwandlung einer $CCl_3$-Gruppe am Phenyl in die -CN-Funktion durch Umsetzung mit $NH_3$ beschrieben.

Es wurde nun ein neues, ökonomischeres und umweltfreundlicheres Verfahren gefunden, das mit überraschend hohen Ausbeuten arbeitet.

Das erfindungsgemässe neue Verfahren zur Herstellung der eingangs definierten 4-Phenyl-pyrrol-derivate der Formel I besteht darin, dass man ein 3-Trifluormethyl-4-phenyl-pyrrol der Formel II

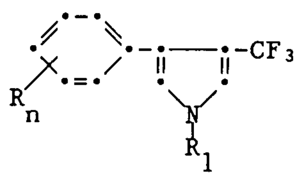

(II),

worin

R für Halogen, $C_1$-$C_6$-Alkyl oder $C_1$-$C_6$-Haloalkyl steht;

n für O, 1 oder 2 steht; und

$R_1$ Wasserstoff oder die Gruppe $C(O)R_2$ repräsentiert, wobei $R_2$ für $C_1$-$C_6$-Alkyl, $C_1$-$C_6$-Haloalkyl, Phenyl oder $C_1$-$C_6$-Alkoxy steht, bei Temperaturen zwischen Raumtmperatur und +230°C und einem Druck von 1 bis 200 bar mit einem Moläquivalent Ammoniak umsetzt.

Als Acylgruppen kommen im allgemeinen alle unsubstituierten oder durch übliche Substituenten (wie Halogen, Cyano, Niederalkoxy, Niederalkylthio, Niederhaloalkyl oder Nitro) substituierten Arylcarbonyl-, Aralkylcarbonyl-, Alkylcarbonyl- und Alkoxycarbonylgruppen in Frage.

Ueberraschenderweise wird bei dieser Reaktion die in 3-Stellung des Pyrrolrings befindliche Trifluormethylgruppe in eine Cyanogruppe übergeführt und gleichzeitig die Acylgruppe am Pyrollstickstoff ($R_1$ = C-(O)$R_2$) abgespalten, so dass man unmittelbar zu den in DE-A-2 927 480 genannten, fungizid aktiven 4-Phenyl-pyrrol-derivaten gelangt, die, sofern N-acetylierte Derivate erwünscht sind, sich auf übliche Weise, z.B. gemäss DE-A-2 927 480 nachacetylieren lassen.

Die erfindungsgemässe Reaktion wird bei Temperaturen zwischen Raumtemperatur und +230°C, insbesondere +160° und +200°C durchgeführt.

Man arbeitet vorzugsweise in einem reaktionsinerten Lösungsmittel oder Lösungsmittelgemisch. Geeignete Lösungsmittel sind z.B.: aliphatische und aromatische Kohlenwasserstoffe wie Benzol, Toluol, Xylole, Petrolether, usw.; Ether und ethereartige Verbindungen wie Dialkylether (Diethylether, Diisopropylether, tert.-Butylmethylether, usw.), Anisol und insbesondere cyclische Ether wie Dioxan oder Tetrahydrofuran; Alkohole wie Alkanole (Methanol, Ethanol, Propanole, Butanole, usw.) und Wasser sowie Gemische derartiger Lösungsmittel untereinander.

Die Umsetzung wird im Druckbereich 1 bis 200 bar durchgeführt; bei Anwesenheit von Wasser vorzugsweise bei 1 bis 50 bar; bei Abwesenheit von Wasser vorzugsweise bei 40 bis 150 bar.

In einer bevorzugten Ausführungsform wird wässriger Ammoniak, vorzugsweise eine konzentrierte wässrige Ammoniaklösung (25 bis 34 Gewischtsprozent $NH_3$) in Dioxan oder Tetrahydrofuran eingesetzt. Man kann den Ammoniak jedoch auch in der Reaktionsmischung z.B. aus einem Ammoniumsalz z.B. einem Ammoniumhalogenid ($NH_4Cl$) und einer Base (z.B. NaOH) erzeugen.

Im allgemeinen wird der Ammoniak im Ueberschuss, mindestens jedoch in äquimolaren Mengen eingesetzt; dabei arbeitet man in üblichen Druckgefässen wie z.B. Bombenrohren, Autoklaven, usw.

Eine besonders bevorzugte Ausführungsform besteht darin, dass man 3-Trifluormethyl-4-(2,3-dichlorphenyl)pyrrol oder ein durch $C(O)R_2$, worin $R_2$ die unter Formel II angegebenen Bedeutungen hat, N-acyliertes Derivat davon bei +50° bis +230°C, vorzugsweise +160° bis +200°C, mit überschüssigem, wässrigen Ammoniak in einem cyclischen Ether, vorzugsweise Dioxan oder Tetrahydrofuran, in einem geschlossenen Druckgefäss zu 3-Cyano-4-(2,3-dichlorphenyl)pyrrol umsetzt.

Die Ausgangssubstanzen der Formel II sind neu, sie sind durch die erfindungsgemässe Umwandlung von $CF_3$ in CN als Ausgangsprodukte zur Herstellung der Verbindungen der Formel I geradezu prädestiniert kund bilden daher einen Gegenstand dieser Erfindung. Sie lassen sich beispielsweise dadurch herstellen, dass man einen Benzaldehyd der Formel III

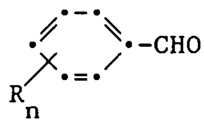

(III)

mit einem Organyloxymethyl-triphenylphosphoniumhalogenid der Formel IV

$$\left[(C_6H_5)_3\overset{\oplus}{P}—CH_2OR_3\right]\ Hal^{\ominus} \qquad (IV)$$

zu einem Styrolderivat der Formel V

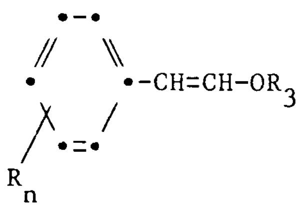

(V)

umsetzt, letzeres in Gegenwart einer Base mit Trifluoressigsäureanhydrid zu einer Verbindung der Formel VI

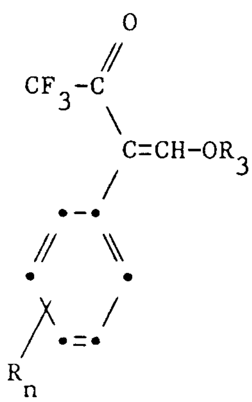

(VI)

acetyliert, (VI) mit einem Alkali-Glycinat, vorzugsweise einem Natrium- oder Kaliumglycinat, in einem Carbonsäureanhydrid der Formel VII

$O[C(O)R_2]_2$ (VII)

zu einem N-acylierten 3-Trifluormethyl-4-phenylpyrrol der Formel II'

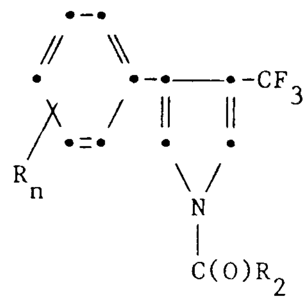

(II')

cyclisiert und, sofern gewünscht, den Acylrest $C(O)R_3$ basisch abspaltet, so dass ein freies 3-Trifluormethyl-4-phenylpyrrol der Formel II"

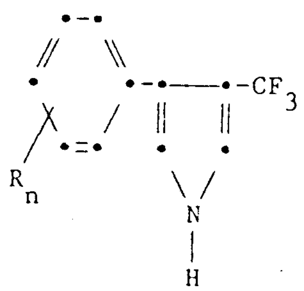

(II")

entsteht, wobei $R_n$ in den Formeln III, V, VI, II' und II" und $R_2$ in den Formeln VII und II' die unter Formel I angegebenen Bedeutungen haben, $R_3$ in Formel IV für einen Organylrest, vorzugsweise einen aromatischen oder aliphatischen Rest, insbesondere für unsubstituiertes oder durch übliche Reste substituiertes [$C_1$-$C_{12}$-Alkyl, Aralkyl oder Phenyl], vor allem $C_1$-$C_6$-Alkyl steht und Hal in Formel IV ein Halogen, vorzugsweise Chlor, Brom oder Jod repräsentiert.

Die Umsetzung des Benzaldehyd III mit dem Organyloxymethyl-triphenylphosphoniumhalogenid IV zu Styrolderivaten der Formel V kann analog zu den in Chem. Ber. 94, 1373 (1961) beschriebenen Reaktionen durchgeführt werden und wird weiter unten an einem Beispiel explizit beschrieben.

Die Benzaldehyde der Formel III, die Organyloxethyl-triphenylphosphoniumhalogenide der Formel IV sowie die Anhydride der Formel VII sind allgemein bekannt oder können analog zu den bekannten Vertretern hergestellt werden.

Dagegen ist die Trifluoracetylierung der Styrole V zu Verbindungen der Formel VI neu. Sie gelingt in hohen Ausbeuten, jedoch im Gegensatz zu herkömmlichen Acetylierungsreaktionen, nur unter Ausschluss von Lösungsmitteln bei Temperaturen zwischen +20° und +150°, vorzugsweise +80° und +120°C und unter erhöhtem Druck. Im allgemeinen arbeitet man mit äquimolaren Mengen oder vorteilhafterweise mit überschüssigem Trifluoressigsäureanhydrid im Bombenrohr, im Autoklaven oder anderen Druckgefässen. Der Einsatz von Säurefreiem Trifluoressigsäureanhydrid ist von Vorteil. Als Basen können bei dieser Reaktion organische und anorganische Basen, vorzugsweise in äquimolarer Menge, eingesetzt werden. Geeignete Basen sind z.B. anorganische Basen wie die Oxide, Hydride, Carbonate und Carbonsäuresalze der Erdalkali-, bevorzugt der Alkalimetalle, insbesondere des Natriums und Kaliums [z.B. NaH, $Na_2CO_3$, $K_2CO_3$, $CaCO_3$, $CH_3COONa$, $C_2H_5COOK$, usw.]. Als organische Basen kommen Trialkylamine, wie z.B. Triethylamin, Piperidin und insbesondere Pyridinbasen wie das freie Pyridin in Betracht. Da die Reaktion von (V) zu (VI) neu ist und zu unmittelbaren Vorprodukten (VI) der fungizid aktiven Verbindungen der Formeln II' und II" führt, stellt sie einen wichtigen Bestandteil der vorliegenden Erfindung dar. Desgleichen bilden die Verbindungen der Formel VI als unmittelbare Vorstufen zu den fungizid aktiven Verbindungen der Formeln II' und II" einen Teil dieser Erfindung.

Die Umsetzung der Verbindung der Formel VI mit einem Alkali-Glycinat und dem Carbonsäureanhydrid VII kann entweder im Eintopfverfahren durchgeführt werden, wobei alle drei Reaktionspartner (VI + Gycinat + VII) gleichzeitig anwesend sind und man auf diese Weise unmittelbar zu Verbindungen der Formel II' gelangt oder, man kann auch zunächst (VI) mit dem Glycinat zu einer isolierbaren Zwischenstufe VIII umsetzen,

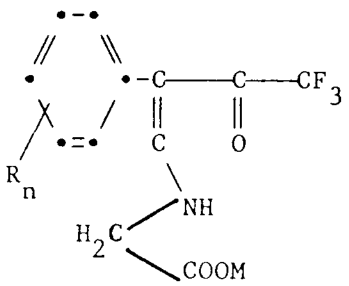

(VIII),

worin $R_n$ wie unter Formel I definiert ist und M für ein Alkalimetallatom, insbesondere Kalium oder Natrium steht, und anschliessend (VIII) durch Reaktion mit dem Carbonsäureanhydrid VII zu Verbindungen der Formel II cyclisieren. Derartige Pyrrolsynthesen sind aus der Literatur bekannt [vgl. Helv. Chem. Acta. 65, 1694 (1982)] und können analog zu den dort beschriebenen Methoden durchgeführt werden.

Die Herstellung von Verbindungen der Formel II'' aus Verbindungen der Formel II' erfolgt durch basische Abspaltung der Acylgruppe $C(O)R_2$. Diese basische Abspaltung kann völlig analog zu literaturbekannten [vgl. Hel.Chem. Acta 65, 407 und 1694 (1982)] Abspaltungen von Schutzgruppen durchgeführt werden. Im allgemeinen arbeitet man bei Temperaturen zwischen -20° und +100°C in üblichen organischen, reaktionsinerten Lösungsmitteln, wobei als Base eine der bereits oben genannten organischen oder anorganischen Basen, vorzugsweise Lithiumaluminiumhydrid, eingesetzt werden kann.

## Herstellung der Vorprodukte

Beispiel V1: Herstellung von

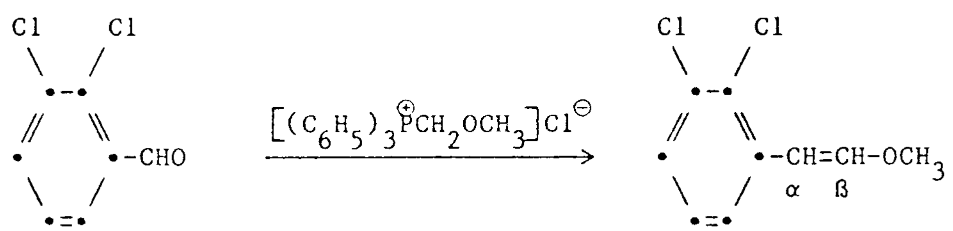


$\beta$-Methoxy-2,3-dichlorstyrol

47,3 g Natriummethylat werden in 1,5 Liter absolutes Ethanol eingetragen. Dann werden zuerst 300,0 g Methoxmethyltriphenylphosphoniumchlorid und danach 142,5 g 2,3-Dichlorbenzaldehyd bei Raumtemperatur portionsweise zugegeben und das Reaktionsgemisch ca. 60 Stunden auf 70° erhitzt. Nach dem Erkalten auf Raumtemperatur wird das Gemisch filtriert und das Filtrat im Vakuum eingeengt. Der Rückstand wird in Pentan aufgenommen, gewaschen, erneut eingeengt und über $SiO_2$ mit Dichlormethan filtriert. Ueberschüssiges Lösungsmittel wird im Vakuum entfernt. Man erhält $\beta$-Methoxy-2,3-dichlorstyrol als 1:1 (E/Z)-Gemisch in Form eines hellgelben Oels.

Beispiel V2: Herstellung von

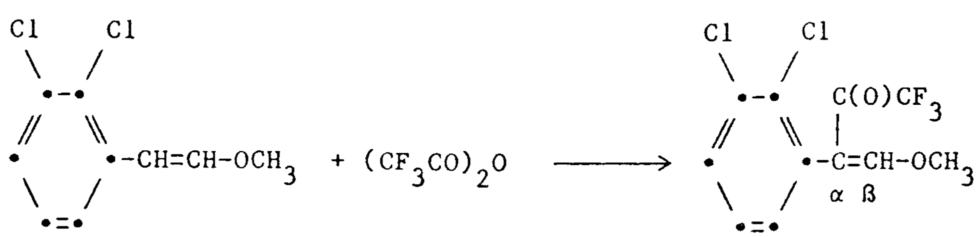

α-Trifluoracetyl-β-methoxy-2,3-dichlorstyrol

13,5 g des oben hergestellten β-Methoxy-2,3-dichlorstyrols werden im Bombenrohr mit 6,1 g Pyridin versetzt. Unter Argon-Schutzgasatmosphäre und unter Kühlen werden zu diesem Gemisch 24,4 g Trifluoressigsäureanhydrid zugetropft. Das Bombenrohr wird verschlossen und 4 Stunden auf 100 ° C erhitzt. Nach Erkalten auf Raumtemperatur wird das Reaktionsgemisch in Dichlormethan aufgenommen und über $SiO_2$ filtriert. Nach Abdampfen des Lösungsmittels erhält man α-Trifluoracetyl-β-methoxy-2,3-dichlorstyrol als gelbes Oel.

Beispiel V3: Herstellung von

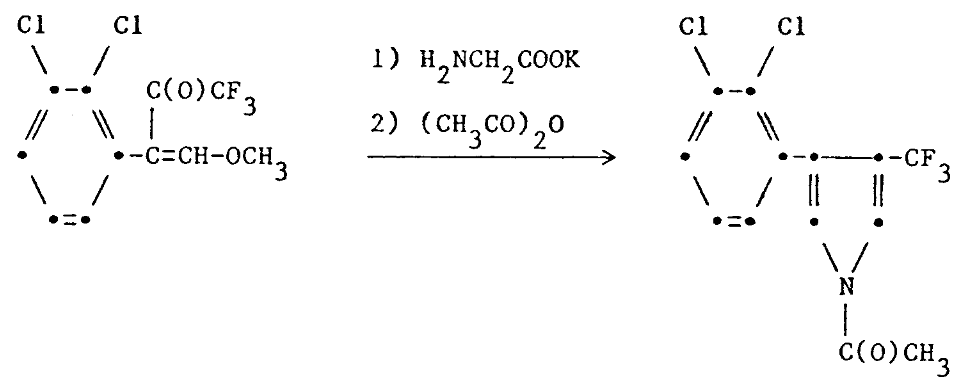


N-Acetyl-4-(2,3-dichlorphenyl)-3-trifluormethylpyrrol

In 160 ml Ethanol werden 2,7 g Glycin und 2,0 g Kaliumhydroxid gelöst und das Gemisch auf 45 ° C erwärmt. Bei dieser Temperatur werden 9,9 g des nach Beispiel V2 hergestellten α-Trifluoracetyl-β-methoxy-2,3-dichlorstyrols zugegeben und das Reaktionsgemisch 90 Minuten unter Rückfluss erhitzt. Anschliessend wird das Gemisch im Vakuum eingeengt, der Rückstand mit 150 ml Essigsäureanhydrid versetzt und die neue Mischung 45 Minuten auf 100 ° C erhitzt. Nach dem Abklingen der $CO_2$-Entwicklung wird das Lösungsmittel durch Eindampfen entfernt, der Rückstand in Toluol aufgenommen und über $SiO_2$ filtriert. Das Filtrat wird erneut eingedampft und der ölige Rückstand mit Hexan überschichtet, wobei N-Acetyl-4-(2,3-dichlorphenyl)-3-trifluormethylpyrrol in Form von beigen Kristallen ausfällt. Smp. 65-66 ° C.

Beispiel V4: Herstellung von

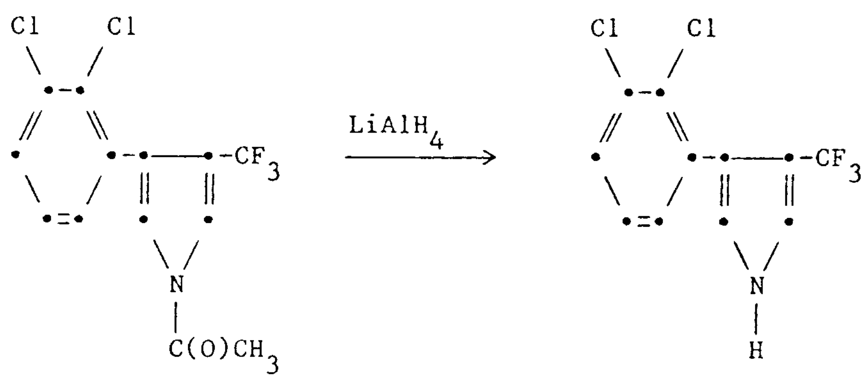


3-Trifluormethyl-4-(2,3-dichlorphenyl)-pyrrol

29,0 g N-Acetyl-4-(2,3-dichlorphenyl)-3-trifluormethylpyrrol werden in 500 ml Diethylether gelöst. Diese Lösung wird auf 0 ° C abgekühlt und portionsweise mit 13,7 g Lithiumaluminiumhydrid versetzt. Anschliessend rührt man noch 1 Stunde. Dann lässt man bei 5° C 55 ml 4 %ige Natronlauge zutropfen, wobei elementarer Wasserstoff entweicht und rührt das Gemisch eine weitere Stunde bei Raumtemperatur. Sodann wird das Gemisch filtriert, das Filtrat über Magnesiumsulfat getrocknet, erneut filtriert und eingeengt. Das resultierende Oel wird bei 150°/8 x $10^{-5}$ mbar destilliert. Aus dem Destillat erhält man durch Ueberschichten mit Hexan 3-Trifluormethyl-4-(2,3-dichlorphenyl)pyrrol in Form von farblosen Kristallen. Smp. 63-65° C.

Analog zu den beschriebenen Arbeitsweisen erhält man auch die nachfolgend genannten, für die vorliegende Erfindung typischen Vertreter von Verbindungen der Formel VI,

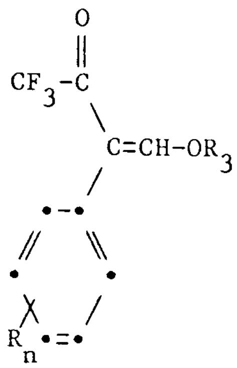

(VI),

wobei die Verbindungen im allgemeinen als 1:1 (E/Z)-Gemische anfallen:

| Verb.Nr. | $R_n$ | $R_3$ |
|---|---|---|
| 1.1 | H | $CH_3$ |
| 1.2 | H | $C_3H_7$-n |
| 1.3 | 3-Cl | $CH_3$ |
| 1.4 | 2-Br | $CH_3$ |
| 1.5 | 2,5-$Cl_2$ | $CH_3$ |
| 1.6 | 2,3-$Cl_2$ | $CH_3$ |
| 1.7 | 2-Cl | $CH_3$ |
| 1.8 | 3-$CF_3$ | $CH_3$ |
| 1.9 | 3-Br | $CH_3$ |
| 1.10 | 3-F | $CH_3$ |
| 1.11 | 3-$CH_3$ | $CH_3$ |
| 1.12 | 4-F | $CH_3$ |
| 1.13 | 4-Cl | $CH_3$ |
| 1.14 | 2,4-$Cl_2$ | $CH_3$ |
| 1.15 | 2,3-$Cl_2$ | $C_2H_5$ |
| 1.16 | 2,3-$Cl_2$ | $C_3H_7$-i |
| 1.17 | 2,3-$Cl_2$ | $C_6H_5$ |
| 1.18 | 2-Cl | $C_6H_5$ |

Analog zu den beschriebenen Arbeitsweisen erhält man auch die nachfolgend aufgeführten, für die vorliegende Erfindung typischen Vertreter der Formel II:

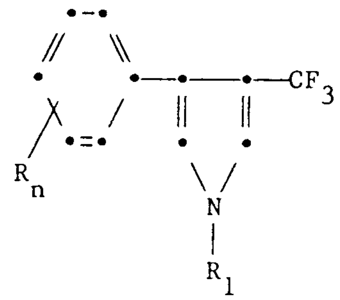

(II)

| Verb.Nr. | $R_n$ | $R_1$ | Physikal. Konstante |
|---|---|---|---|
| 2.1 | 2,3-$Cl_2$ | H | Smp. 63-65°C |
| 2.2 | 2-Br | H | |
| 2.3 | 2,5-$Cl_2$ | H | |
| 2.4 | 2-Cl | H | Smp. 93-94°C |
| 2.5 | 2-$CF_3$ | H | |
| 2.6 | 3-Br | H | |
| 2.7 | 3-F | H | |
| 2.8 | 3-$CH_3$ | H | |
| 2.9 | 4-F | H | |
| 2.10 | 4-Cl | H | |
| 2.11 | 2,4-$Cl_2$ | H | |
| 2.12 | 3-Cl | H | |
| 2.13 | H | H | |
| 2.14 | 2,3-$Cl_2$ | C(O)$CH_3$ | Smp. 65-66°C |
| 2.15 | 2-Br | C(O)$CH_3$ | |
| 2.16 | 2,5-$Cl_2$ | C(O)$CH_3$ | |

| Verb.Nr. | $R_n$ | $R_1$ | Physikal. Konstante |
|---|---|---|---|
| 2.17 | 2-Cl | $C(O)CH_3$ | Sdp. 130-140°/ 0,01 mbar |
| 2.18 | $2-CF_3$ | $C(O)CH_3$ | |
| 2.19 | 3-Br | $C(O)CH_3$ | |
| 2.20 | 3-F | $C(O)CH_3$ | |
| 2.21 | $3-CH_3$ | $C(O)CH_3$ | |
| 2.22 | 4-F | $C(O)CH_3$ | |
| 2.23 | 4-Cl | $C(O)CH_3$ | |
| 2.24 | $2,4-Cl_2$ | $C(O)CH_3$ | |
| 2.25 | 3-Cl | $C(O)CH_3$ | |
| 2.26 | $2,3-Cl_2$ | $C(O)C_2H_5$ | |
| 2.27 | $2,3-Cl_2$ | $C(O)C_3H_7$-i | |
| 2.28 | $2,3-Cl_2$ | $C(O)C_6H_5$ | |
| 2.29 | $2,3-Cl_2$ | $C(O)OCH_3$ | |
| 2.30 | 3-Cl | $C(O)OC_2H_5$ | |
| 2.31 | 3-Cl | $C(O)C_6H_5$ | |
| 2.32 | 3-Cl | $C(O)CF_3$ | |
| 2.33 | 2-Cl | $C(O)C_4H_9$-n | |
| 2.34 | 2-Cl | $C(O)OC_3H_7$-n | |
| 2.35 | 2-Cl | $C(O)C_2H_5$ | |
| 2.36 | 2,3-Cl | $C(O)CF_3$ | |
| 2.37 | H | $C(O)CH_3$ | Sdp. 110-120°/ 0,01 mbar |

Herstellung der Endprodukte:

Beispiel E1: Herstellung von

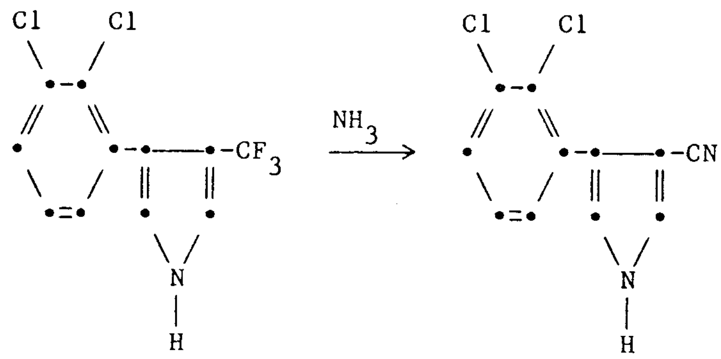


4-(2,3-Dichlorphenyl)3-cyanopyrrol

4,0 g 3-Trifluormethyl-4-(2,3-dichlorphenyl)pyrrol, 21 ml 25 %iger wässriger Ammoniak und 40 ml Dioxan werden 26 Stunden bei 180 ° C im Autoklaven gerührt. Nach Abkühlen auf Raumtemperatur wird das Reaktionsgemisch filtriert und das klare Filtrat eingedampft. Der Rückstand wird in Ethylacetat aufgenommen, mit Wasser und anschliessend mit verdünnter Natriumchloridlösung gewaschen, über Magnesiumsulfat getrocknet, filtriert und eingedampft. Der Rückstand wird durch Zugabe von Hexan zur Kristallisation gebracht, abfiltriert und getrocknet. Man erhält 3,4 g (100 % der Theorie) 4-(2,3-Dichlorphenyl)-3-cyanopyrrol. Smp. 150 ° C.

Beispiel E2: Herstellung von

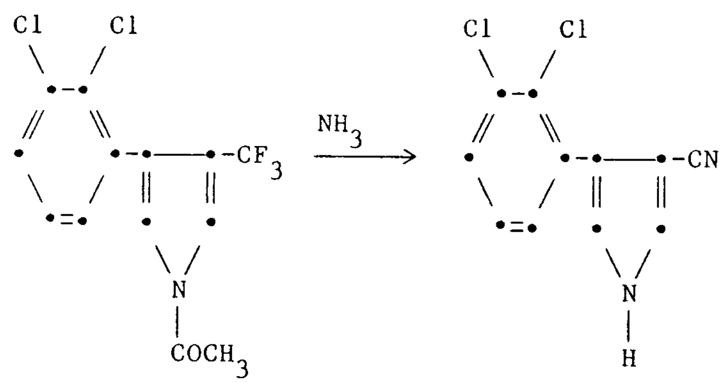


4-(2,3-Dichlorphenyl)-3-cyanopyrrol

6,4 g N-Acetyl-4-(2,3-dichlorphenyl)-3-trifluormethylpyrrol, 15 ml 25 %iger wässrige Ammoniaklösung und 60 ml Dioxan werden 18 Stunden bei 160 ° C im Autoklaven gerührt. Das Reaktionsgemisch wird auf Raumtemperatur abgekühlt, filtriert und das klare Filtrat eingedampft. Der zähe Rückstand wird in Ethylacetat gelöst, zuerst mit Wasser, dann mit verdünnter Natriumchloridlösung gewaschen, über Natriumsulfat getrocknet, filtriert und eingedampft. Der Rückstand wird mit n-Hexan versetzt, und die ausgefallenen Kristalle werden abfiltriert und getrocknet. Man erhält 5,3 g 4-(2,3-Dichlorphenyl)-3-cyanopyrrol. Smp. 149-150 ° C.

Analog zu den beschriebenen Arbeitsweisen werden auch die nachfolgenden Verbindungen der Formel I hergestellt:

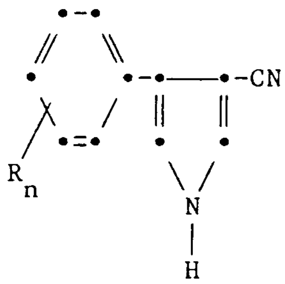

(I)

| Verb. Nr. | $R_n$ | Smp. ° C |
|---|---|---|
| 1 | 2,3-$Cl_2$ | 149-150 |
| 2 | 2-Br | 135-138 |
| 3 | 2,5-$Cl_2$ | 137-142 |
| 4 | 2-Cl | 136-138 |
| 5 | 3-$CF_3$ | 87-89 |
| 6 | 3-Br | 132-134 |
| 7 | 3-F | 138-139 |
| 8 | 3-$CH_3$ | 109-111 |
| 9 | 4-F | 137-139 |
| 10 | 4-Cl | 153-155 |
| 11 | 2,4-$Cl_2$ | 150-152 |
| 12 | 3-Cl | 138-140 |
| 13 | H | 120-123 |

**Patentansprüche**

**1.** Verfahren zur Herstellung von 4-Phenyl-pyrrolderivaten der Formel I,

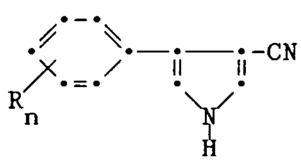

(I),

worin
R für Halogen, $C_1$-$C_6$-Alkyl oder $C_1$-$C_6$-Haloalkyl steht; und n für 0, 1 oder 2 steht; dadurch gekennzeichnet, dass man ein 3-Trifluormethyl-4-phenyl-pyrrol der Formel II,

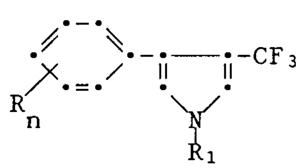

(II),

worin
$R_n$ wie unter Formel I definiert ist und $R_1$ Wasserstoff oder die Gruppe $C(O)R_2$ repräsentiert, wobei $R_2$ für $C_1$-$C_6$-Alkyl, $C_1$-$C_6$-Haloalkyl, Phenyl oder $C_1$-$C_6$-Alkoxy steht, bei Temperaturen zwischen Raumtemperatur und +230 ° C und bei einem Druck von 1 bis 200 bar mit mindestens einem Moläquivalent Ammoniak umsetzt.

2. Verfahren nach Anspruch 1, dadurch gekennzeichnet, dass man die Reaktion bei Temperaturen zwischen +160° und 200°C durchführt.

3. Verfahren nach einem der Ansprüche 1 bis 2, dadurch gekennzeichnet, dass man die Reaktion in einem reaktionsinerten Lösungsmittel durchführt.

4. Verfahren nach Anspruch 3, dadurch gekennzeichnet, dass man als Lösungsmittel aliphatische oder aromatische Kohlenwasserstoffe, Ether oder etherartige Verbindungen, Alkohole, Wasser oder Gemische derartiger Verbindungen verwendet.

5. Verfahren nach Anspruch 4, dadurch gekennzeichnet, dass man als Ether cyclische Ether einsetzt.

6. Verfahren nach Anspruch 5, dadurch gekennzeichnet, dass man als cyclische Ether Dioxan oder Tetrahydrofuran einsetzt.

7. Verfahren nach einem der Ansprüche 1 bis 6, dadurch gekennzeichnet, dass man bei Anwesenheit von Wasser die Reaktion bei einem Druck von 1 bis 50 bar durchführt.

8. Verfahren nach einem der Ansprüche 1 bis 6, dadurch gekennzeichnet, dass man die Reaktion bei Abwesenheit von Wasser bei einem Druck von 40 bis 150 bar durchführt.

9. Verfahren nach einem der Ansprüche 1 bis 6, dadurch gekennzeichnet, dass man als Agens eine wässrige Ammoniaklösung einsetzt.

10. Verfahren nach Anspruch 9, dadurch gekennzeichnet, dass man als Agens eine konzentrierte wässrige Ammoniaklösung einsetzt.

11. Verfahren nach Anspruch 9, dadurch gekennzeichnet, dass die wässrige Lösung 25 bis 34 Gewichtsprozent Ammoniak enthält.

12. Verfahren nach einem der Ansprüche 1 bis 11, dadurch gekennzeichnet, dass man die Umsetzung in einem Druckgefäss durchführt.

13. Verfahren nach einem der Ansprüche 1 bis 6 und 8, dadurch gekennzeichnet, dass man wasserfreies Ammoniak einsetzt.

14. Verfahren nach Anspruch 13, dadurch gekennzeichnet, dass man das Ammoniak im Reaktionsgemisch aus einem Ammoniumsalz und einer Base freisetzt.

15. Verfahren nach Anspruch 1, dadurch gekennzeichnet, dass man 3-Trifluormethyl-4-(2,3-dichlorphenyl)-pyrrol oder ein durch $C(O)R_2$, worin $R_2$ die unter Formel II angegebenen Bedeutungen hat, N-acyliertes Derivat davon bei +50° bis +230°C mit überschüssigem, wässrigen Ammoniak in einem cyclischen Ether in einem geschlossenen Druckgefäss zu 3-Cyano-4-(2,3-dichlorphenyl)pyrrol umsetzt.

16. Verfahren zur Herstellung der Verbindungen gemäss der in Anspruch 1 definierten Formel I, dadurch gekennzeichnet, dass man einen Benzaldehyd der Formel III

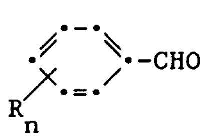

(III)

mit einem $C_1$-$C_6$-Alkoxymethyl-triphenylphosphoniumhalogenid der Formel IV

$$\left[ (C_6H_5)_3\overset{\oplus}{P}-CH_2OR_3 \right] Hal^{\ominus} \qquad (IV)$$

zu einem Styrolderivat der Formel V

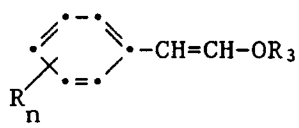

(V)

umsetzt, letzteres ohne Lösungsmittel bei Temperaturen zwischen +20 °C und +150 °C und unter erhöhtem Druck in Gegenwart einer Base mit Trifluoressigsäureanhydrid zu einer Verbindung der Formel VI

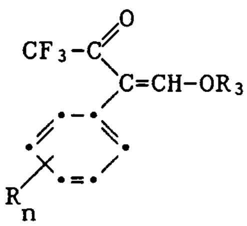

(VI)

acetyliert, (VI) mit einem Alkali-Glycinat in einem Carbonsäureanhydrid der Formel VII

$O[C(O)R_2]_2$ (VII)

zu einem N-acylierten 3-Trifluormethyl-4-phenylpyrrol der Formel II'

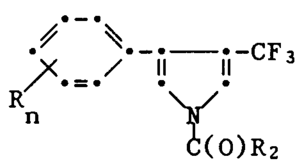

(II')

cyclisiert und letzteres in die Verbindungen der Formel I überführt, wobei $R_n$ in den Formeln III, V, VI, II' und $R_2$ in den Formeln VII und II' die unter Formel I angegebenen Bedeutungen haben, $R_3$ in den Formeln IV, V und VI für $C_1$-$C_6$-Alkyl steht und Hal in Formel IV Chlor, Brom oder Jod repräsentiert.

**17.** Die in Anspruch 1 definierten Verbindungen der Formel II.

**18.** Verbindungen der Formel II nach Anspruch 17, worin R für Chlor steht, n 1 oder 2 bedeutet und $R_1$ die in Anspruch 1 angegebenen Bedeutungen repräsentiert.

## Claims

**1.** A process for preparing a 4-phenylpyrrole derivative of the formula I

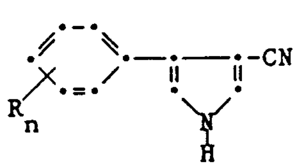

(I)

in which R is halogen, $C_1$-$C_6$alkyl or $C_1$-$C_6$haloalkyl and n is 0, 1 or 2, which comprises reacting a 3-trifluoromethyl-4-phenylpyrrole of the formula II

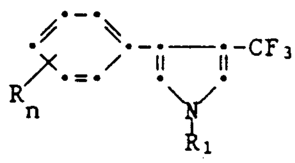

(II)

in which $R_n$ is as defined under formula I and $R_1$ is hydrogen or the group $C(O)R_2$, in which $R_2$ is $C_1$-$C_6$alkyl, $C_1$-$C_6$haloalkyl, phenyl or $C_1$-$C_6$-alkoxy, at temperatures between room temperature and +230 ° C and at a pressure of 1 to 200 bar with at least one mole equivalent of ammonia.

**2.** A process according to claim 1, wherein the reaction is performed at temperatures of between +160 ° and 200 ° C.

**3.** A process according to either of claims 1 and 2, wherein the reaction is performed in a solvent inert to the reaction conditions.

**4.** A process according to claim 3, wherein the solvent used is an aliphatic or aromatic hydrocarbon, an ether or ethereal compound, an alcohol, water, or a mixture of such compounds.

**5.** A process according to claim 4, wherein the ether used is a cyclic ether.

**6.** A process according to claim 5, wherein the cyclic ether used is dioxane or tetrahydrofuran.

**7.** A process according to any one of claims 1 to 6, wherein the reaction is performed at a pressure of 1 to 50 bar in the presence of water.

**8.** A process according to any one of claims 1 to 6, wherein the reaction is performed at a pressure of 40 to 150 bar in the absence of water.

**9.** A process according to any one of claims 1 to 6, wherein the agent used is an aqueous ammonia solution.

**10.** A process according to claim 9, wherein the agent used is a concentrated aqueous ammonia solution.

**11.** A process according to claim 9, wherein the aqueous solution contains 25 to 34 per cent by weight of ammonia.

**12.** A process according to any one of claims 1 to 11, wherein the reaction is performed in a pressure vessel.

**13.** A process according to any one of claims 1 to 6 and 8, wherein anhydrous ammonia is used.

**14.** A process according to claim 13, wherein the ammonia is released from an ammonium salt and a base in the reaction mixture.

**15.** A process according to claim 1, which comprises reacting 3-trifluoromethyl-4-(2,3-dichlorophenyl)-pyrrole, or a derivative thereof N-acylated by $C(O)R_2$, in which $R_2$ has the meanings defined under formula II, at +50 ° to +230 ° C with excess aqueous ammonia in a cyclic ether, in a closed pressure vessel, to obtain 3-cyano-4-(2,3-dichlorophenyl)pyrrole.

**16.** A process for preparing a compound of the formula I defined in claim 1, which comprises reacting a benzaldehyde of the formula III

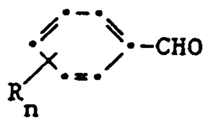

(III)

with a $C_1$-$C_6$ alkoxymethyltriphenylphosphonium halide of the formula IV

$$\left[(C_6H_5)_3\overset{\oplus}{P}\text{-}CH_2OR_3\right] \quad Hal^{\ominus} \qquad (IV)$$

to obtain a styrene derivative of the formula V

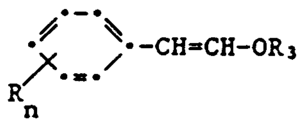

(V),

acetylating the latter in the presence of a base without a solvent at temperatures between +20 °C and +150 °C and under elevated pressure with trifluoroacetic anhydride to obtain a compound of the formula VI

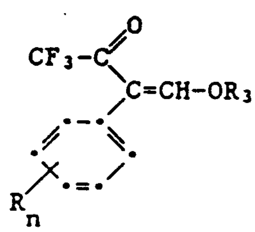

(VI),

cyclising (VI) with an alkali metal glycinate in a carboxylic anhydride of the formula VII

$O[C(O)R_2]_2$ (VII)

to obtain an N-acylated 3-trifluoromethyl-4-phenylpyrrole of the formula II'

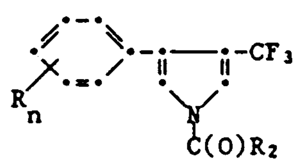

(II')

and converting the latter into a compound of the formula I, $R_n$ in the formulae III, V, VI and II' and $R_2$ in the formulae VII and II' having the meanings defined under formula I, $R_3$ in the formulae IV, V and VI being $C_1$-$C_6$ alkyl, and Hal in formula IV being chlorine, bromine or iodine.

**17.** A compound of the formula II as defined in claim 1.

**18.** A compound of the formula II according to claim 17, in which R is chlorine, n is 1 or 2 and $R_1$ has one

of the meanings defined in claim 1.

**Revendications**

1. Procédé de préparation de dérivés du 4-phényl-pyrrole de formule I

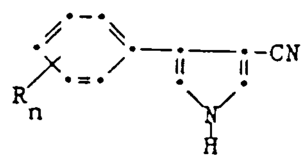

(I),

dans laquelle
R représente un halogène, un groupe alkyle en C 1-C 6 ou halogénoalkyle en C 1-C 6 ; et n est égal à 0, 1 ou 2 ; caractérisé en ce que l'on fait réagir un 3-trifluorométhyl-4-phényl-pyrrole de formule II

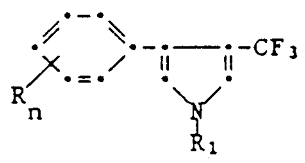

(II),

dans laquelle
$R_n$ a les significations indiquées en référence à la formule I et $R_1$ représente l'hydrogène ou le groupe $C(O)R_2$ dans lequel $R_2$ représente un groupe alkyle en C 1-C 6, halogénoalkyle en C 1-C 6, phényle ou alcoxy en C 1-C 6, avec au moins un équivalent molaire d'ammoniac à des températures comprises entre la température ambiante et +230 °C et sous une pression de 1 à 200 bar.

2. Procédé selon la revendication 1, caractérisé en ce que l'on effectue la réaction à des températures de +160 à 200 °C.

3. Procédé selon l'une des revendications 1 à 2, caractérisé en ce que l'on effectue la réaction dans un solvant inerte dans cette réaction.

4. Procédé selon la revendication 3, caractérisé en ce que l'on utilise en tant que solvants des hydrocarbures aliphatiques ou aromatiques, des éthers ou composés apparentés, des alcools, l'eau ou des mélanges de ces solvants.

5. Procédé selon la revendication 4, caractérisé en ce que les éthers utilisés sont des éthers cycliques.

6. Procédé selon la revendication 5, caractérisé en ce que l'éther cyclique utilisé est le dioxanne ou le tétrahydrofuranne.

7. Procédé selon l'une des revendications 1 à 6, caractérisé en ce que, en présence d'eau, on effectue la réaction sous une pression de 1 à 50 bar.

8. Procédé selon l'une des revendications 1 à 6, caractérisé en ce que, en l'absence d'eau, on effectue la réaction sous une pression de 40 à 150 bar.

9. procédé selon l'une des revendications 1 à 6, caractérisé en ce que l'ammoniac mis en oeuvre est à l'état d'ammoniaque.

10. Procédé selon la revendication 9, caractérisé en ce que l'ammoniac mis en oeuvre consiste en ammoniaque concentrée.

11. Procédé selon la revendication 9, caractérisé en ce que l'ammoniaque est à une concentration de 25 à

34% en poids d'ammoniac.

**12.** Procédé selon l'une des revendications 1 à 11, caractérisé en ce que l'on effectue la réaction dans un récipient résistant à la pression.

**13.** Procédé selon l'une des revendications 1 à 6 et 8, caractérisé en ce que l'on utilise de l'ammoniac anhydre.

**14.** Procédé selon la revendication 13, caractérisé en ce que l'on produit l'ammoniac dans le mélange de réaction à partir d'un sel d'ammonium et d'une base.

**15.** Procédé selon la revendication 1, caractérisé en ce que l'on fait réagir le 3-trifluorométhyl-4-(2,3-dichlorophényl)-pyrrole ou un dérivé de ce composé acylé à l'azote par un groupe $C(O)R_2$ dans lequel $R_2$ a les significations indiquées en référence à la formule II, à des températures de +50 à +230 °C, avec un excès d'ammoniaque dans un éther cyclique, dans un récipient fermé résistant à la pression, la réaction donnant le 3-cyano-4-(2,3-dichlorophényl)-pyrrole.

**16.** Procédé pour la préparation des composés de formule I de la revendication 1, caractérisé en ce que l'on fait réagir un benzaldéhyde de formule III

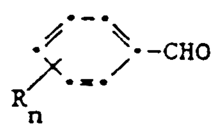

(III)

avec un halogénure d'(alcoxy en C 1-C 6)-méthyl-triphénylphosphonium de formule IV

$$\left[ (C_6H_5)_3\overset{\oplus}{P}-CH_2OR_3 \right] Hal^{\ominus} \qquad (IV)$$

la réaction donnant un dérivé du styrène de formule V

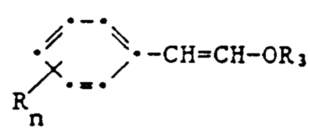

(V)

qu'on convertit par acétylation à l'aide de l'anhydride trifluoracétique, sans solvant, à des températures allant de +20 à +150 °C et sous pression, en un composé de formule VI

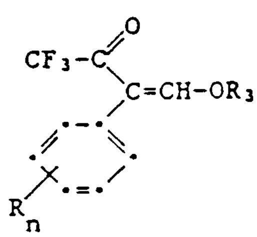

(VI)

qu'on cyclise par un glycinate alcalin dans un anhydride d'acide carboxylique de formule VII

$O[C(O)R_2]_2$ (VII)

en un 3-trifluorométhyl-4-phényl-pyrrole N-acylé de formule II'

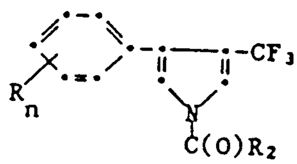

(II')

qu'on convertit en le composé de formule I, $R_n$ ayant dans les formules III, V, VI, II' et $R_2$ dans les formules VII et II' les significations indiquées en référence à la formule I, $R_3$ représentant dans les formules IV, V et VI un groupe alkyle en C 1-C 6 et Hal, dans la formule IV, le chlore, le brome ou l'iode.

**17.** Les composés de formule II définis dans la revendication 1.

**18.** Composés de formule II de la revendication 17 dans laquelle R représente le chlore, n est égal à 1 ou 2 et $R_1$ a les significations indiquées dans la revendication 1.